# DEMANDE DE BREVET EUROPEEN

(11) **EP 3 342 307 A1**
(43) Date de publication de la demande: **04.07.2018**
(21) Numéro de dépôt: 17210435.8
(22) Date de dépôt: 22.12.2017
(51) Int. Cl.: A45C 11/00, A45C 13/02

(54) **ACCESSOIRE POUR DISPOSITIF ELECTRONIQUE DE TYPE TELEPHONE MOBILE**

(30) Priorité: 23.12.2016 BE 201605971
(71) Demandeur: SUREST SPRL, 1180 Bruxelles (BE)
(72) Inventeur: PLATTARD, Régis, B-1180 Bruxelles (BE)
(74) Mandataire: Pronovem

(57) **Abrégé**

Accessoire (1) pour dispositif électronique (2) de type téléphone mobile comprenant :
un emplacement ou élément (3) conformé pour se positionner ou se placer autour ou sur de le dispositif électronique (2) et comprenant des moyens pour fixer ledit dispositif électronique (2) dans ou sur ledit emplacement ou élément (3), ledit emplacement ou élément (3) comprenant une paroi de fond (4), et éventuellement une ou plusieurs parois périphériques (5, 6, 7 ou 8) s'étendant perpendiculairement au plan formé par ladite paroi de fond (4),
et un compartiment (10) avec un espace de stockage séparé de l'emplacement ou élément (3) disposé parallèlement à ladite paroi de fond (4) et à l'arrière dudit emplacement ou élément (3) lorsque le dispositif électronique est positionné ou placé, ledit compartiment (10) étant délimité par la paroi de fond (4) de l'emplacement ou élément (3) ou par une paroi parallèle à ladite paroi de fond (4) et au moins quatre parois périphériques (12, 12, 14, 15) ainsi qu'une paroi (16) permettant de créer le compartiment (10) de stockage, ainsi que des moyens d'ouverture/fermeture dudit compartiment (10) permettant de passer d'une position d'ouverture à une position de fermeture et inversement,
caractérisé en ce que l'accessoire comprend des moyens de dissipation de la chaleur émise par ledit dispositif électronique (2) lorsqu'il est positionné ou placé dans ledit emplacement ou élément (3).

## Description

### Objet de l'invention

La présente invention se rapporte à un accessoire pour un dispositif électronique tel un téléphone mobile et plus particulièrement un dispositif servant de protection pour ledit téléphone mobile et plus particulièrement le smartphone.

### Etat de la technique

Pour les objets quotidiens les plus fragiles, transportés et manipulés plusieurs fois par jour, comme par exemple des lunettes, mais également et surtout des appareils électroniques portatifs, il est bien connu d'utiliser des dispositifs de protection, comme par exemple une housse, rigide ou souple, une coque ou un étui, afin de protéger ces objets contre l'usure normale due à leur utilisation, mais également contre les chocs, des frottements, et les protéger de la poussière ou de l'humidité.

En particulier pour les appareils électroniques, tels les téléphones mobiles ou les Smartphones, il est bien connu de les protéger par un étui en tissu ou en cuir souple. Néanmoins, une telle solution n'est pas pratique car elle ne permet pas de manipuler le téléphone sans devoir le sortir de son étui.

Est également connu, l'emploi d'une coque rigide, ou d'une demi-coque, comprenant une paroi formant un fond, de forme et de dimensions adaptées au téléphone à protéger, une paroi qui est entourée sur son pourtour par au moins deux parois formant des bords s'étendant sensiblement de façon perpendiculaire au plan formé par la paroi formant le fond, pour former une cavité dans laquelle prend place le téléphone mobile. Généralement, la coque ou la demi-coque comprend également des moyens d'accrochage du téléphone pour engagement réversible du téléphone dans la coque ou demi-coque. Classiquement, cet engagement se fait par force, les parois des bords étant suffisamment souples pour s'écarter lors de l'engagement, et suffisamment résilientes pour maintenir serrés les bords du téléphone, comme par exemple comme décrit dans le document US20130264235. Par ailleurs, la coque peut comprendre un rabat, plus souple que la coque, pouvant se rabattre sur la face du téléphone non engagé dans la demi-coque, qui comprend généralement l'écran du téléphone.

Par ailleurs, outre leur fonction de protection, il a été proposé des dispositifs de protection, en particulier pour téléphones mobiles, qui soient multifonction. Ainsi, il existe des étuis de protection de téléphone comprenant un étui à lunettes, comme décrit dans le document US20110210018, ou un étui faisant office de portefeuille, comme décrit dans US2013118923, qui décrit un étui comprenant un compartiment arrière, pouvant recevoir notamment des billets de banque ou des cartes de crédits, et accessible par exemple par une fente ouverte.

Le document US20140251368 décrit, quant à lui, une demi-coque de protection d'un téléphone mobile comprenant en partie arrière un compartiment accessible par une fente, mais pouvant également être accessible par le glissement longitudinal de la paroi extérieure du compartiment ou à l'aide d'une paroi battante articulée à l'aide de moyens faisant charnière. Ce dispositif est destiné à recevoir des objets courants tels que des cartes de crédit, cartes ID, cartes SIM ou mémoire, clés ou encore de l'argent. Tous ces objets peuvent être maintenus dans le compartiment sans précaution particulière.

Les solutions existantes, comprenant une paroi mobile fermant le compartiment arrière des coques ou demi-coques, présentent l'inconvénient de ne pas permettre l'ouverture du compartiment d'une seule main, en particulier à l'aide de la seule main tenant le téléphone mobile.

De plus, comme tous les appareils électroniques portatifs, un téléphone mobile est susceptible de chauffer, car les composants électroniques qui le composent, y compris la batterie en phase de charge, peuvent atteindre des températures relativement élevées, même lors d'une utilisation normale, induisant ainsi une montée en température de l'ensemble de l'appareil. De plus, l'utilisation d'un dispositif de protection n'arrange rien, mais aggrave encore plus le phénomène car une coque ou un étui présente l'inconvénient de réduire, voire empêcher, le bon refroidissement par ventilation de l'appareil électronique en question, et d'empêcher le fonctionnement de moyens de dissipation de chaleur quand ils existent.

Par ailleurs, pour ce qui concerne les dispositifs de protection comprenant un compartiment arrière, la chaleur de l'appareil électronique portatif, en particulier d'un téléphone, se propage au contenu dudit compartiment arrière. Ainsi, de tels dispositifs de protection ne sont pas adaptés pour le stockage de produits sensibles à la chaleur, comme par exemple des médicaments.

Le document US 2015/328411 a quant à lui proposé de combiner un téléphone portable avec les fonctions d'un dispositif d'injection cutanée d'un produit liquide tel que de l'insuline. Dans ce cas, des mesures spécifiques mais relativement simples de protection du liquide contre l'échauffement ont été proposées.

### Buts de l'invention

La présente invention vise à fournir une alternative aux solutions de l'état de la technique existantes.

La présente invention vise à fournir un dispositif pouvant servir comme accessoire d'un dispositif ou appareil électronique qui ne présente pas les inconvénients de l'état de la technique.

La présente invention vise également à fournir un dispositif servant d'accessoire pour dispositif électronique, comprenant un compartiment de stockage qui soit facile d'accès, et pouvant en particulier être manipulé d'une main, avantageusement la main tenant le dispositif électronique.

La présente invention vise également à fournir une solution pour protéger les produits stockés, en particulier les médicaments, dans le compartiment du dispositif de l'invention de la chaleur émanant de l'appareil électronique.

### Résumé de l'invention

La présente invention se rapporte à un dispositif constitué par un accessoire pour dispositif électronique de type téléphone mobile comprenant :
un emplacement ou élément conformé pour se positionner ou se placer autour ou sur le dispositif électronique et comprenant des moyens pour fixer ledit dispositif électronique dans ou sur ledit emplacement ou élément, ledit emplacement ou élément comprenant au moins une paroi de fond, et éventuellement une ou plusieurs parois périphériques s'étendant perpendiculairement au plan formé par ladite paroi de fond,
et un compartiment avec un espace de stockage séparé de l'emplacement ou élément disposé parallèlement à ladite paroi de fond et à l'arrière dudit emplacement ou élément lorsque le dispositif électronique est positionné ou placé, ledit compartiment étant délimité par la paroi de fond de l'emplacement ou élément ou par une paroi parallèle à ladite paroi de fond et au moins quatre parois périphériques ainsi qu'une paroi mobile permettant de créer le compartiment de stockage, des moyens d'ouverture/fermeture dudit compartiment permettant de passer d'une position d'ouverture à une position de fermeture et inversement, et des moyens d'isolation thermique associés à des moyens de dissipation de la chaleur émise par ledit dispositif électronique lorsqu'il est positionné ou placé dans ou sur ledit emplacement ou élément.

Selon des modes préférés de l'invention, le dispositif de l'invention comprend au moins une, ou une combinaison quelconque appropriée, des caractéristiques suivantes :
- des moyens réfléchissant les rayonnements UV sont prévus ;
- les moyens de dissipation de la chaleur sont, ou comprennent, une plaque de métal faisant office de vecteur thermique ;
- les moyens de dissipation de la chaleur sont ou comprennent une plaque de métal ayant une conductibilité thermique supérieure à 20, de préférence 100, de préférence 200 Watt par mètre - Kelvin ;
- les moyens réfléchissant les rayonnements UV sont au moins partiellement constitués par une surface au moins partiellement plane de la plaque de métal faisant office de vecteur thermique tandis que les moyens de dissipation de la chaleur sont constitués par l'autre surface de la plaque de métal faisant office de vecteur thermique ;
- la plaque de métal est au moins en partie en contact direct avec l'air libre par une de ses surfaces ;
- la plaque de métal faisant office de vecteur thermique présente une structure 3D de manière à augmenter sa surface de dissipation ;
- la surface de dissipation de la plaque de métal doit être de 30%, de préférence 40%, de préférence 50%, de préférence 100% supérieur à la surface propre de la plaque de métal ;
- la plaque de métal présente une épaisseur comprise entre 1 et 3 mm ;
- le matériau de la plaque de métal est un matériau conducteur, de préférence de l'aluminium, le cuivre, l'argent ou tout autre matériau ayant des propriétés de conduction de la chaleur ou un alliage de ceux-ci ;
- la plaque de métal présente des orifices qui présentent une surface inférieure à 10% par rapport à la surface propre de la plaque de métal ;
- la proportion de la surface plane par rapport à la surface propre de la plaque de métal est supérieure à 50 %, de préférence supérieure à 75 % de préférence supérieure à 90 % ;
- la plaque de métal n'est pas directement en contact avec la paroi de fond et/ou avec une paroi parallèle à la paroi de fond de manière à créer un espace servant de lame d'air et constituant un moyen d'isolation thermique ;
- L'épaisseur de la lame d'air est d'au moins 1 mm ;
- Les moyens d'ouverture/fermeture dudit compartiment permettent le glissement de la paroi mobile, transversalement par rapport à l'emplacement ou élément, dans un plan sensiblement parallèle au plan formé par la paroi de fond de l'emplacement ou élément ;
- les moyens d'ouverture/fermeture du compartiment comprennent au moins un ressort dont l'une des extrémités est fixée à la paroi mobile et l'autre extrémité est fixée soit à la paroi de fond ou à la paroi parallèle à ladite paroi de fond, soit directement et de préférence sur la plaque de métal ;
- il comprend en outre des moyens de verrouillage du compartiment ;
- il comprend en outre des moyens d'authentification afin de ne permettre l'accès au contenu du compartiment qu'à une ou des personnes préalablement déterminées comme étant autorisés à accéder audit contenu ;
- il comprend en outre des comprimés d'un ou plusieurs médicaments disposés dans le compartiment et des moyens d'observance et/ou des moyens de suivi de l'observance d'un traitement médical ;
- les moyens d'observance et/ou des moyens de suivi de l'observance d'un traitement médical coopèrent avec des moyens de verrouillage de l'emplacement ou élément et/ou des moyens de détection de l'ouverture dudit compartiment.
- le mécanisme d'ouverture comporte un système à double pression pour ouvrir l'appareil qui permet de d'empêcher l'accès au contenu, par exemple les médicaments, par des petites mains non autorisées, par exemple des enfants.

La présente invention porte aussi sur un téléphone mobile ou un dispositif électronique équipé du dispositif servant d'accessoire selon l'invention.

La présente invention porte également sur l'utilisation de l'accessoire selon l'invention pour équiper un téléphone mobile et pour stocker des comprimés d'un ou plusieurs médicaments.

Selon un mode particulier de réalisation de l'invention, l'accessoire selon l'invention est utilisé pour l'observance et/ou le suivi de l'observance d'un traitement médical.

### Brève description des figures

La figure 1 est une représentation schématique d'une vue de perspective d'un mode de réalisation particulier de l'invention dans lequel un téléphone mobile, du type smartphone, est équipé du dispositif servant d'accessoire selon l'invention.
La figure 2 est une représentation schématique d'une vue de côté du mode de réalisation représenté à la figure 1.
La figure 3 est une représentation schématique d'une vue en perspective du mode de réalisation de la figure 1, dans laquelle le compartiment de stockage du dispositif servant d'accessoire selon l'invention est en position ouverte.
La figure 4 est une représentation schématique d'une vue de l'avant du mode de réalisation représenté à la figure 3.
La figure 5 est une représentation schématique d'une vue de l'arrière du mode de réalisation représenté à la figure 3.
La figure 6 est une représentation schématique d'une vue en perspective et partiellement éclatée du mode de réalisation tel que représenté à la figure 1.
La figure 7 est une représentation schématique d'une vue éclatée du mode de réalisation représenté à la figure 1.
La figure 8 est une représentation schématique d'une partie essentielle constituée par une plaque métallique servant de moyen de dissipation de chaleur du dispositif servant d'accessoire selon l'invention.
La figure 9 décrit une vue en coupe transversale de la paroi de fond et de la lame d'air et de la plaque métallique embossée selon une forme d'exécution préférée.

### Description détaillée d'une ou plusieurs formes d'exécution

Le dispositif servant d'accessoire selon l'invention va maintenant être décrit, à titre d'exemple, dans un mode de réalisation particulier dans lequel l'accessoire 1 a la forme d'une demi-coque et le dispositif électronique 2 est un smartphone. Toutefois, il est à noter que la description de cet exemple est faite à titre d'illustration de l'invention sans avoir de caractère limitatif. En effet, outre le fait qu'il puisse s'agir d'une demi-coque de protection, l'accessoire selon l'invention peut également être un étui, une housse ou un élément à apposer sur le dispositif électronique ou une enveloppe, souple, rigide ou semi-rigide, enveloppant tout ou partie de le dispositif électronique à protéger.

Par ailleurs, le dispositif électronique 2, pouvant être équipé et protégé par l'accessoire 1 selon l'invention, peut également être par exemple un assistant numérique personnel, un téléphone mobile, une tablette, une phablette, un ordinateur portable, une console de jeu, un assistant d'aide à la conduite, un appareil de photographie numérique, un lecteur mobile audio et/ou vidéo.

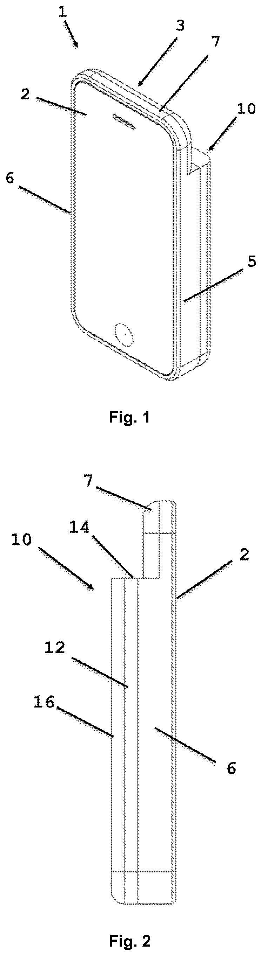

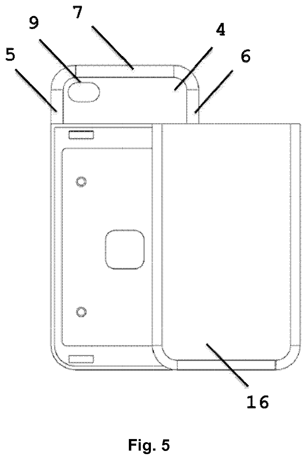

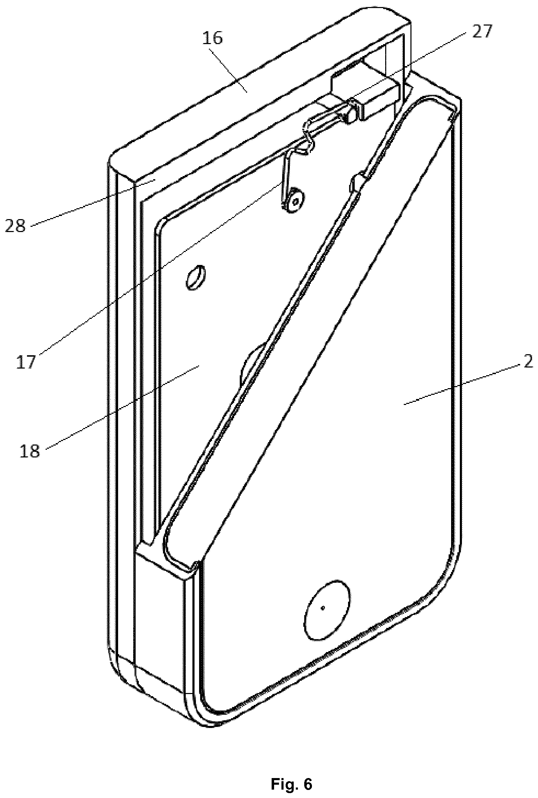

Ainsi, comme représenté dans les figures 1 à 9, l'accessoire 1 selon l'invention est une demi-coque, de forme et de dimensions compatible avec, et afin de recevoir de façon réversible, tout ou partie d'un dispositif électronique, en particulier un téléphone mobile 2. De préférence, s'agissant de recevoir un smartphone, l'accessoire 1 selon l'invention a une forme parallélépipédique.

L'accessoire 1 comprend un emplacement ou élément 3, destiné à recevoir le téléphone mobile 2, formé par au moins une première paroi 4 dont une face forme le fond de l'emplacement ou élément et de préférence au moins deux parois 5 et 6 s'étendant dans un plan sensiblement perpendiculaire au plan formé par la première paroi 4, pour former au moins deux parois périphériques opposées l'une à l'autre, formant ainsi les bords longitudinaux ou les bords latéraux dudit emplacement ou élément 3. De préférence, l'emplacement ou élément 3 comprend quatre parois périphériques 5, 6, 7 et 8, formant ainsi des bords longitudinaux et des bords latéraux.

L'accessoire 1 comprend tous moyens adéquats pour maintenir le téléphone mobile 2 dans l'emplacement ou élément 3. De préférence, ces moyens sont, ou comprennent, les parois périphériques 5, 6 ou 5, 6, 7 et 8 formant les bords longitudinaux et/ou latéraux, qui sont suffisamment souples pour s'écarter lors de l'engagement du téléphone mobile 2, et suffisamment résilientes pour maintenir serrés les bords du téléphone mobile 2 une fois celui-ci en place dans l'accessoire 1, la surface frontale du téléphone mobile 2 étant accessible à l'utilisateur, tandis que la surface arrière fait face à, ou est au contact avec, la paroi de fond 4 de l'emplacement ou élément 3. On peut envisager que l'accessoire 1 soit simplement fixé par collage, par soudure ou clipsage au dispositif électronique.

De préférence, la paroi de fond 4 et/ou les parois périphériques 5, 6 ou 5, 6, 7 et 8 de l'emplacement ou élément 3 comprennent une ou plusieurs ouvertures 9 faisant face à, et ayant une forme et des dimensions adéquates avec, des moyens de commandes et/ou de connectique du téléphone mobile 2 et/ou un microphone, un ou plusieurs haut-parleurs et/ou un ou plusieurs moyens de prise de vue éventuellement présents sur l'une ou l'autre des faces et/ou des bords du téléphone mobile 2.

L'accessoire 1 comprend en outre un compartiment 10 qui est un compartiment de stockage, disposé à l'arrière de l'emplacement ou élément 3 de l'accessoire 1. Ce compartiment 10 est couplé à l'emplacement ou élément 3 de toutes manières adéquates. De préférence, une partie du compartiment 10 fait partie intégrante de l'emplacement ou élément 3.

De préférence, le compartiment 10 est formé d'une part par une première paroi de fond 11, ou par la seconde surface de la paroi de fond 4 de l'emplacement ou élément 3 de l'accessoire 1, d'autre part par au moins quatre parois périphériques 12, 13, 14, 15 et par une paroi mobile 16 qui sert adéquatement de couvercle. Soit les quatre parois périphériques 12, 13, 14, 15 s'étendent, à partir de la paroi de fond 11, dans un plan sensiblement perpendiculaire au plan formé par ladite paroi de fond 11, ou bien elles s'étendent à partir de la paroi mobile 16, dans un plan sensiblement perpendiculaire au plan formé par ladite paroi mobile 16.

Selon l'invention l'accessoire 1 comprend des moyens de dissipation 18 de la chaleur émise par le téléphone mobile 2 ou le dispositif correspondant positionné dans l'emplacement ou élément 3 associés à des moyens d'isolation.

Les parois de l'emplacement ou élément 3 et du compartiment 10 comprennent, ou sont formées, de tous matériaux adéquats. De préférence, elles sont faites de plastique et/ou de métal. De préférence l'ensemble sera réalisé en plastique, à l'exception des moyens de dissipation de chaleur.

De préférence, les moyens de dissipation 18 de chaleur sont disposés sur, ou intégrés dans, la paroi de fond 4 de l'emplacement ou élément 3. Néanmoins, ils peuvent également être disposés sur, ou intégrés dans, la paroi de fond 13 du compartiment 10.

Selon un mode de réalisation préféré, les moyens de dissipation 18 de la chaleur constitués par une plaque métallique et ne seront pas apposés en contact direct avec la paroi de fond 13, mais seront placés à une distance de celle-ci de telle manière à permettre la présence d'une lame d'air 19 servant de moyens d'isolation.

De préférence, des moyens réfléchissant les rayons UV sont également prévus.

De préférence, les moyens de dissipation de la chaleur comprennent un, ou sont constitués d'un, ventilateur, avantageusement alimenté électriquement par les moyens d'alimentation électrique du téléphone mobile 2.

Dans un autre mode de réalisation de l'invention, les moyens de dissipation de la chaleur sont, ou comprennent, une plaque de métal 18, de préférence une plaque texturée, ou 3D, agissant comme vecteur de dissipation thermique.

De préférence, les moyens de dissipation de la chaleur sont réalisés à l'aide d'une plaque métallique présentent deux surfaces, d'une part (18a et 18b) et d'autre part (18c et 18d), la première surface (18c et 18d) étant au moins partiellement plane (18c) va servir de moyens réfléchissant les rayonnements UV, tandis que l'autre surface (18a et 18b) est de préférence expansée en 3D et va servir de moyen de dissipation de la chaleur.

De préférence, la plaque s'étend hors de l'accessoire 1, et est, en contact, au moins en partie avec l'air libre, extérieur, ce qui permet de servir de fluide de convection.

L'utilisation d'une plaque en métal 18 comme vecteur de dissipation et dispersion de chaleur présente en outre l'avantage de rigidifier l'ensemble de l'accessoire 1 selon l'invention, une rigidité qui présente également l'avantage de permettre une ouverture et une fermeture précise et fiable du compartiment 10, en permettant de guider le l'ouverture du compartiment 10.

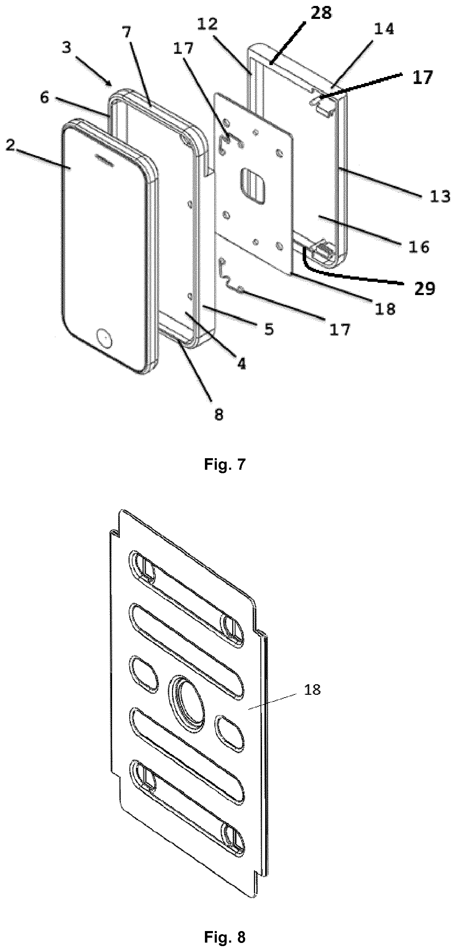

De préférence, la plaque en métal 18 ainsi que représentée à la figure 8 présente une forme 3D ou structurée de manière à augmenter la surface de dissipation.

Avantageusement, cette plaque peut être obtenue par embossage, ce qui va lui conférer une structure 3D avec des creux (18d) sur une de ses 2 faces et des bosses ou collines (18a) sur l'autre face. La partie en creux sera entourée de parties planes servant de moyens de réflexion du rayonnement UV, tandis que la surface avec les bosses ou collines permet d'augmenter la surface de dissipation de la chaleur.

Idéalement, la surface de dissipation de la chaleur est de préférence 30%, préférence 40%, préférence 50%, préférence 100%, à la surface propre de la plaque métallique.

De même, le rapport entre la surface plane et la surface propre de la plaque de métal est supérieure à 50% est supérieure à 75%, est supérieure à 90%.

Par surface propre, on entend la surface projetée sur une perpendiculaire à la direction d'embossage, c'est-à-dire qu'elle correspond à la surface totale non emboutie.

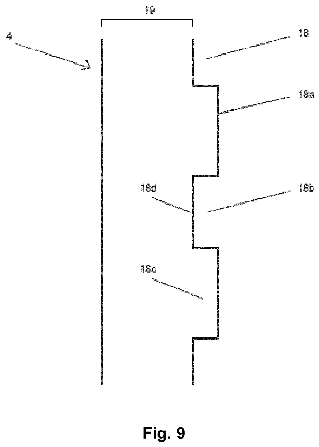

La figure 9 montre la succession des couches selon une coupe transversale de l'accessoire de l'invention.

On y observe de manière avantageuse, que dans le cas d'un dépôt d'une plaquette ou blister directement sur la plaque métallique celle-ci ne sera en contact qu'avec quelques points (hauts) de la plaque métallique, c'est-à-dire sur les points les plus emboutis (18a). Cela évite de transférer des calories à ladite plaque ou blister.

Pour que le compartiment 10 passe d'une position ouverte à une position fermée, et inversement, soit il est mobile, par rapport à l'emplacement ou élément 3, par un mouvement de rotation ou de translation, soit seulement la paroi mobile 16 est mobile en rotation ou en translation par rapport à l'emplacement ou élément 3.

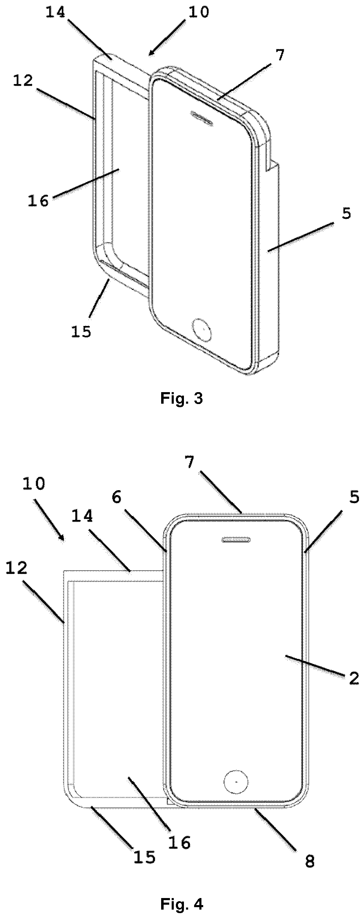

Dans un mode de réalisation particulier de l'invention (figures 3 à 5), le compartiment 10 coulisse, en particulier la paroi mobile 16 et ses parois périphériques 12, 13, 14, 15, coulissent, transversalement par rapport à l'emplacement ou élément 3, dans un plan sensiblement parallèle au plan formé par la paroi de fond 4 de l'emplacement ou élément 3, sur l'un et/ou l'autre des bords longitudinaux de l'emplacement ou élément 3 pour une utilisation par un usager qui soit droitier ou gaucher, ou pour une utilisation ambidextre de l'accessoire 1 selon l'invention.

Le compartiment 10 peut passer d'une position fermée à une position ouverte, et inversement, par une manipulation de la paroi mobile 16, ou de l'ensemble du compartiment 10 de l'accessoire 1, à l'aide d'une seule main, en particulier à l'aide de celle tenant l'accessoire 1 selon l'invention, sans avoir besoin de maintenir l'emplacement ou élément 3, ou la paroi mobile 16, de l'autre main.

Ainsi, à titre d'exemple, et pour l'accessoire 1 ayant l'emplacement ou élément 3 faisant face à l'utilisateur, le compartiment 10 de l'accessoire 1 peut être ouvert par l'action du pousse sur le bord de l'emplacement ou élément 3, ou la surface du téléphone mobile 2 disposé dans l'accessoire 1, dans un mouvement transversal, dans un plan sensiblement parallèle au plan formé par paroi mobile 16, éventuellement aidé par une pression du bout des doigts sur le bord de la paroi mobile 16 ou du compartiment 10 dans le sens opposé, pour que, par exemple le compartiment 10 s'ouvre vers la paume de la main de l'utilisateur.

La fermeture du compartiment 10 peut alors se faire par une pression du pouce sur le bord de la paroi mobile 16 ou du compartiment 10 ou par la pression des bouts des doigts sur le bord de l'emplacement ou élément 3.

Le compartiment 10 de l'accessoire 1 selon l'invention, faisant face cette fois-ci à l'utilisateur, peut également être ouvert par l'action du pousse de la main sur le bord de la paroi mobile 16 ou du compartiment 10, éventuellement aidé par une pression dans le sens inverse du bout des doigts sur le bord de l'emplacement ou élément 3, la fermeture pouvant se faire par une pression du pouce sur le bord de l'emplacement ou élément 3, éventuellement aidée par une pression en sens inverse du bout des doigts sur le bord de la paroi mobile 16 ou du compartiment 10.

De préférence, l'ouverture et la fermeture à une main du compartiment 10 s'effectue à l'aide de tous moyens adéquats. Il peut, par exemple, s'agir de moyens comprenant au moins un guide engageant au moins un rail pour permettre le glissement de la paroi mobile 16, ou, comme représenté à la figure 4, des moyens comprenant au moins un ressort 17 dont l'une des extrémités est fixée sur la paroi de fond 11 du compartiment 10, ou une plaque intermédiaire 18 disposée sur la paroi de fond 11 et éventuellement engageant les parois périphériques 12, 13, 14, 15, et dont l'autre extrémité est fixée sur la plaque mobile 16 de préférence un ergot 27 prévu à cet effet. De préférence, il y a deux ressorts 17 qui avantageusement sont des ressorts en zigzag.

Avantageusement, le compartiment doit pouvoir s'ouvrir/ou se fermer tout en ayant la possibilité de lire l'écran du téléphone ou dispositif électronique.

De préférence, le compartiment 10 a une forme et/ou des dimensions adéquates qui est et/ou sont fonction du contenu qu'il est censé renfermé, et de préférence, fonction de la forme et/ou des dimensions de l'emplacement ou élément 3 auquel il est couplé, et éventuellement des ouvertures 9 pratiquées dans ledit emplacement ou élément 3 au regard des moyens de commandes et/ou de connectique du téléphone mobile 2 et/ou un microphone, un ou plusieurs haut-parleurs et/ou un ou plusieurs moyens de prise de vue éventuellement présents sur l'une ou l'autre des faces et/ou des bords du téléphone mobile 2, afin de ne pas les recouvrir. Dans un mode de réalisation particulier, les bords longitudinaux 12 et 13, et éventuellement un ou les deux bords latéraux 14 ou 15, du compartiment 10 sont sensiblement alignés avec les bords correspondants de l'emplacement ou élément 3 permettant ainsi, au compartiment 10, d'avoir un large débattement d'ouverture et un accès aisé au contenu du compartiment 10.

Avantageusement, l'ouverture 9 sert d'ouverture pour la caméra et sera de préférence teintée ou matifiée afin d'éviter les reflets du flash. Ceci permet d'éviter une surexposition, le cas échéant, des photos. Il est bien entendu également que la partie mobile et surtout la paroi mobile 16 ne recouvre pas l'ouverture 9.

De préférence, la paroi mobile 16 peut être extensible, ce qui présente l'avantage de pouvoir accroire le volume de stockage du compartiment 10.

De préférence, l'accessoire 1 comprend des moyens de verrouillage du compartiment 10. De préférence, ces moyens sont commandés ou contrôlés par des moyens logiciels mis en oeuvre par le téléphone mobile 2 disposé dans l'accessoire 1 selon l'invention.

En effet, avantageusement, les bordures de la plaque métallique 17 peuvent servir de guides à des glissières 28 et 29 présentes dans la partie mobile 16 servant de couvercle.

De préférence, les moyens de verrouillage coopèrent avec des moyens d'authentification permettant l'accès au contenu du compartiment 10 à la personne ou aux personnes déterminées comme étant autorisées, ces moyens d'authentification pouvant être disposés sur la surface externe de la paroi mobile 16 du compartiment 10 ou se trouvant sous la forme de moyens logiciels mise en oeuvre par le téléphone mobile 2 disposé dans l'accessoire 1 selon l'invention.

De préférence, le compartiment 10 est destiné à recevoir, ou comprendre, des moyens de payement comprenant, ou étant, une ou plusieurs cartes de crédit, billets de banque, pièces de monnaie, ou bien encore des clés ou des moyens de maquillage.

Dans un autre mode de réalisation, le compartiment 10 est destiné à recevoir, ou comprendre, des sucreries, par exemple des bonbons.

Dans un autre mode de réalisation, le compartiment 10 est destiné à recevoir, ou comprendre, des comprimés, ou des pilules, sous une forme individualisée ou sous la forme de plaquettes ou de blisters. Les termes « comprimés » et « pilules » couvrent toute forme galénique adéquate pour une absorption orale, par exemple d'un médicament, de vitamines ou de compléments alimentaires. Dans ce mode de réalisation, le compartiment 10 peut comprendre des parois de séparation afin d'individualiser les comprimés ou les pilules, ce qui présente l'avantage d'organiser la prise d'un traitement médicamenteux.

Accessoirement, l'invention pourra-t-être utilisée pour améliorer le suivi des traitements des animaux notamment domestiques. Le produit pourra-t-être alors avantageusement commercialisé par un groupement de vétérinaires par exemple.

Dans ce dernier mode de réalisation, l'accessoire 1 selon l'invention peut également comprendre des moyens d'observance et/ou des moyens de suivi de l'observance d'un traitement médical, comprenant de préférence des moyens matériels et/ou logiciels, qui peuvent être regroupés au sein d'un dispositif couplé par tous moyens adéquats, ou faisant partie intégrante, de la paroi mobile 16 du compartiment 10.

Dans un autre mode de réalisation de l'invention, ces moyens d'observance et/ou des moyens de suivi de l'observance d'un traitement médical sont des moyens logiciels mis en oeuvre par le téléphone mobile 2.

De préférence, les moyens d'observance du traitement médical génèrent et diffuse un ou plusieurs messages sonores ou visuels afin d'alerter l'usager qu'il est temps de prendre un comprimé, ou de recharger le compartiment 10 en comprimés, par exemple au travers du haut-parleur et/ de l'écran et/ou du vibreur du téléphone mobile 2.

De préférence, l'accessoire 1 comprend des moyens de verrouillage du compartiment 10 qui coopèrent avec les moyens d'observance d'un traitement médical pour ne permettre l'accès à l'intérieur du compartiment 10, et donc au(x) comprimé(s), selon un plan, ou planning, prédéterminé de traitement médical. De préférence, l'accessoire 1 ne permet que l'accès qu'à un comprimé, ou pilule, à la fois.

De préférence, les moyens d'observance du traitement médical peuvent comprendre, ou interagir, avec de moyens de commande auprès d'un pharmacien ou d'un médecin du ou des médicaments présents dans le compartiment 10, des moyens étant de préférence des moyens logiciels mise en oeuvre par le téléphone mobile 2.

De préférence, les moyens de suivi de l'observance du traitement médical permettent de savoir si l'utilisateur de l'accessoire 1 selon l'invention a bien accédé au compartiment 10 après qu'une alerte ait été émise. Ces moyens peuvent, de préférence, également effectuer des analyses statistiques sur l'observance du traitement en fonction des accès au contenu du compartiment 10 en fonction du plan prédéterminé de traitement médical.

De préférence, les moyens de suivi de l'observance du traitement médical coopèrent avec les moyens de verrouillage et/ou des moyens de détection de l'ouverture du compartiment 10, par exemple par l'intermédiaire d'un contacteur, afin d'enregistrer l'ouverture dudit compartiment 10, et donc la prise d'un médicament.

De préférence, les moyens de suivi de l'observance du traitement médical comprennent des moyens de comptage de l'ouverture du compartiment 10 et permet de renseigner l'utilisateur sur le nombre de comprimés restant dans le compartiment 10.

De préférence, les moyens de suivi de l'observance du traitement médical comprennent, ou coopèrent avec, des moyens de génération d'un rapport de suivi du traitement médical, un rapport à destination et/ou communiqué à un médecin et/ou pharmacien, sa communication étant réalisée de préférence par le téléphone mobile 2.

## Revendications

1. Accessoire (1) pour dispositif électronique (2) de type téléphone mobile comprenant :
un emplacement ou élément (3) conformé pour se positionner ou se placer autour ou sur le dispositif électronique (2) et comprenant des moyens pour fixer ledit dispositif électronique (2) dans ou sur ledit emplacement ou élément (3), ledit emplacement ou élément (3) comprenant au moins une paroi de fond (4), et éventuellement une ou plusieurs parois périphériques (5, 6, 7 ou 8) s'étendant perpendiculairement au plan formé par ladite paroi de fond (4),
et un compartiment (10) avec un espace de stockage séparé de l'emplacement ou élément (3) disposé parallèlement à ladite paroi de fond (4) et à l'arrière dudit emplacement ou élément (3) lorsque le dispositif électronique est positionné ou placé, ledit compartiment (10) étant délimité par la paroi de fond (4) de l'emplacement ou élément (3) ou par une paroi parallèle à ladite paroi de fond (4) et au moins quatre parois périphériques (12, 12, 14, 15) ainsi qu'une paroi mobile (16) permettant de créer le compartiment (10) de stockage, ainsi que des moyens d'ouverture/fermeture dudit compartiment (10) permettant de passer d'une position d'ouverture à une position de fermeture et inversement,
**caractérisé en ce que** l'accessoire comprend des moyens d'isolation thermique associés à des moyens de dissipation de la chaleur émise par ledit dispositif électronique (2) lorsqu'il est positionné ou placé dans ledit emplacement ou élément (3).

2. Accessoire (1) selon la revendication 1, **caractérisé en ce que** des moyens réfléchissant les rayonnements UV sont prévus.

3. Accessoire (1) selon la revendication 1 ou 2, dans lequel les moyens de dissipation de la chaleur sont, ou comprennent, une plaque de métal (18) faisant office de vecteur thermique.

4. Accessoire (1) selon l'une quelconque des revendications précédentes, **caractérisé en ce que** les moyens de dissipation de la chaleur sont ou comprennent une plaque de métal ayant une conductibilité thermique supérieure à 20, de préférence 100, de préférence 200 Watt par mètre - Kelvin.

5. Accessoire (1) selon l'une quelconque des revendications précédentes, **caractérisé en ce que** les moyens réfléchissant les rayonnements UV sont au moins partiellement constitués par une surface au moins partiellement plane de la plaque de métal (18) faisant office de vecteur thermique tandis que les moyens de dissipation de la chaleur sont constitués par l'autre surface de la plaque de métal (18) faisant office de vecteur thermique.

6. Accessoire (1) selon la revendication 1 ou 2, dans lequel la plaque de métal (18) est au moins en partie en contact direct avec l'air libre par une de ses surfaces.

7. Accessoire (1) selon l'une quelconque des revendications précédentes, **caractérisé en ce que** la plaque de métal (18) faisant office de vecteur thermique présente une structure 3D de manière à augmenter sa surface de dissipation.

8. Accessoire (1) selon l'une quelconque des revendications précédentes, **caractérisé en ce que** la surface de dissipation de la plaque de métal doit être de 30%, de préférence 40%, de préférence 50%, de préférence 100% supérieur à la surface propre de la plaque de métal.

9. Accessoire (1) selon l'une quelconque des revendications précédentes, **caractérisé en ce que** la plaque de métal présente une épaisseur comprise entre 1 et 3 mm.

10. Accessoire (1) selon l'une quelconque des revendications précédentes, **caractérisé en ce que** le matériau de la plaque de métal(18) est un matériau conducteur, de préférence de l'aluminium, le cuivre, l'argent ou tout autre matériau ayant des propriétés de conduction de la chaleur ou un alliage de ceux-ci.

11. Accessoire (1) selon l'une quelconque des revendications précédentes, **caractérisé en ce que** la plaque de métal présente des orifices qui présentent une surface inférieure à 10% par rapport à la surface propre de la plaque de métal.

12. Accessoire (1) selon l'une quelconque des revendications précédentes, **caractérisé en ce que** la proportion de la surface plane par rapport à la surface propre de la plaque de métal (18) est supérieure à 50 %, de préférence supérieure à 75 % de préférence supérieure à 90 %.

13. Accessoire (1) selon l'une quelconque des revendications précédentes, **caractérisé en ce que** la plaque de métal (18) n'est pas directement en contact avec la paroi de fond et/ou avec une paroi parallèle à la paroi de fond de manière à créer un espace servant de lame d'air et constituant un moyen d'isolation thermique.

14. Accessoire (1) selon la revendication 13, **caractérisé en ce que** l'épaisseur de la lame d'air est de au moins 1mm.

15. Utilisation de l'accessoire (1) selon l'une quelconque des revendications précédentes pour stocker des objets tels des comprimés, des cartes de crédits, objets de petite taille.

16. Utilisation de l'accessoire (1) selon l'une quelconque des revendications précédentes, en coopération avec un dispositif électronique de type téléphone mobile pour l'observance et/ou le suivi de l'observance d'un traitement médical.
